# EUROPEAN PATENT APPLICATION

(11) **EP 4 572 033 A1**
(43) Date of publication of application: **18.06.2025**
(21) Application number: 24218956.1
(22) Date of filing: 11.12.2024
(51) Int. Cl.: H01R 13/631, A61M 16/18, G06F 1/26, H01R 13/00, H01R 13/648, H01R 27/02

(54) **CONNECTOR FOR MEDICAL DEVICE, CONNECTOR ASSEMBLY, AND ANESTHESIA MACHINE SYSTEM**

(30) Priority: 15.12.2023 CN 202311736729
(71) Applicant: GE Precision Healthcare LLC, Waukesha, WI 53188 (US)
(72) Inventor: JI, Yueting, Wuxi, 214028 (CN); DING, Chao, Wuxi, 214028 (CN); BAI, Yongjie, Wuxi, 214028 (CN); FANG, Hui, Wuxi, 214028 (CN); LIU, Yiqiang, Wuxi, 214028 (CN); GILLING, Michael, Madison, 53718 (US)
(74) Representative: Fennell, Gareth Charles

(57) **Abstract**

The present invention relates to a connector for a medical device, a connector assembly, and an anesthesia machine system. The connector for a medical device includes: a first housing, including a first end and a second end opposite each other; a second housing, the second end of the first housing being configured to be floatingly connected within the second housing, and the first end being exposed out of the second housing; and terminals, provided on the first end of the first housing, the terminals including a power supply terminal and a signal terminal.

## Description

### TECHNICAL FIELD

The present invention relates to the field of medical devices, and more specifically, to a connector for a medical device, a connector assembly including the connector, and an anesthesia machine system including the connector assembly.

### BACKGROUND

Connectors are commonly used important apparatuses for connecting two devices, or two parts of a device. The connectors may be used for connection of medical devices. Inside some connectors for medical devices, one end of a terminal of the connector is soldered on a circuit board and extends from one side of the circuit board by a certain length. These terminals are generally used for power connections of medical devices. For example, a component to be powered in a medical device may have a port matching the connector described above, and after the port is connected to the connector, power can be supplied to the component to be powered via the terminal.

The connector facilitates the detachable connection of the component to be powered in the medical device, and especially in some use scenarios, the component to be powered can be frequently separated from or connected to the connector according to actual usage requirements. The inventor found that the connector and the port are easily damaged during the process of connection and separation, because the connector, the port, and electrical elements therein may be bent and damaged if the angle of connection is somewhat inaccurate. Moreover, in addition to being powered, components to be powered may need to be communicatively connected. In this case, an additional communication interface is required. This is disadvantageous for compact components. In addition, the additional interface increases the complexity of operation.

### SUMMARY

In order to solve at least one or a plurality of the technical problems described above and/or other possible technical problems, provided in the present invention is a connector for a medical device, comprising: a first housing, comprising a first end and a second end opposite each other; a second housing, the second end of the first housing being configured to be floatingly connected within the second housing, and the first end being exposed out of the second housing; and terminals, provided on the first end of the first housing, the terminals comprising a power supply terminal and a signal terminal.

Preferably, the lengths of at least some signal terminals exposed at the first end are less than the length of the power supply terminal exposed at the first end.

Preferably, the at least some signal terminals comprise a test terminal, the test terminal being configured to control, according to the electrical connection status thereof, energization and deenergization of the power supply terminal, and the length difference causing electrical connection of the power supply terminal to be earlier than electrical connection of the test terminal, and causing removal of the electrical connection of the power supply terminal to be later than removal of the electrical connection of the test terminal.

Preferably, the signal terminals comprise: a CAN bus terminal, configured to transmit a signal to a medical device connected to the connector; and/or an Ethernet terminal, configured to provide an Ethernet connection to the connected medical device.

Preferably, the first housing comprises a first portion and a second portion, and the first portion and the second portion are different in at least one among shape or size.

Preferably, the power supply terminals comprise at least two power supply terminals, and the terminals further comprise at least two power return terminals, wherein each of the power supply terminals corresponds to each of the power return terminals to form a power supply loop.

Preferably, the terminals further comprise a ground terminal.

Preferably, the connector further comprises a circuit board accommodated in the first housing, the terminals being fixed on a first side of the circuit board and passing through the first end of the first housing, and a second side of the circuit board comprising a plurality of electric wires coupled to the terminals, wherein the plurality of electric wires are divided into at least two cables.

Preferably, the electric wires in each of the at least two cables are bundled via a bundling member.

Preferably, a first end of the electric wire close to the terminals is fixed in the first housing, and a second end of the electric wire located away from the terminals is fixed in the second housing.

Preferably, the connector further comprises: a first wrapping member, provided in the first housing, and configured to gather the first ends of the electric wires together, and fix the first ends of the electric wires in the first housing via glue; and a second wrapping member, provided in the second housing, and configured to gather the second ends of the electric wires together, and fix the second ends of the electric wires in the second housing via a fastener.

Preferably, a gap is present between the first housing and the second housing to allow for circumferential floating of the first housing relative to the second housing, and a protrusion is provided on a side wall of the first housing, a notch being provided at a position on a side wall of the second housing opposite the protrusion, the notch accommodating the protrusion, and the notch being larger than the protrusion, thereby at least partially defining the floating range of the first housing relative to the second housing.

Preferably, the second end of the first housing comprises a guide pin extending from the second end, the second housing comprising a hole for accommodating the guide pin, and the connector further comprising a spring, and the spring being sleeved on the guide pin of the first housing and being accommodated in the hole of the second housing, so as to assist the first housing in axial floating relative to the second housing.

Preferably, the radial dimension of a base of the guide pin is greater than the radial dimension of the spring, and the interior of the hole comprises a step for abutting against the spring to restrict the spring in a particular space.

Also provided in the present invention is a connector assembly, comprising: the connector according to any one of the above paragraphs, and an electronic evaporator, comprising an interface matching the connector, wherein the electronic evaporator performs power and signal transmission via the terminals of the connector.

Preferably, a beveled surface is provided around an inner surface of the interface of the electronic evaporator, and when contacting the beveled surface, the first end of the first housing can float to guide the interface to be connected to the connector.

Preferably, the terminals of the connector comprise pogo pins, and the interface of the electronic evaporator comprise pads in one-to-one correspondence with the pogo pins.

Also provided in the present invention is an anesthesia machine system, comprising: an anesthesia machine main unit; and the connector assembly according to any one of the above paragraphs, wherein the anesthesia machine main unit is configured to supply power to the electronic evaporator via the power supply terminal of the connector, and achieve signal communication with the electronic evaporator via the signal terminal.

Preferably, the anesthesia machine main unit comprises a power source for supplying power to the power supply terminal of the connector and a power management circuit, and the power management circuit is configured to: when a test signal generated by connection between the test terminal among the signal terminals and the interface of the electronic evaporator is detected, control the power source to supply power to the power supply terminal of the connector; and when the test signal is not detected, control the power source to stop supplying power to the power supply terminal of the connector.

Preferably, the power management circuit is further configured to: after the test signal is detected and lasts a certain time, control the power source to supply power to the power supply terminal; and when the test signal is not detected, immediately stop the power source supplying power to the power supply terminal.

Preferably, the anesthesia machine system further comprises a chassis, and the second housing of the one or more connectors can be fixed to the chassis, wherein the bottom of the chassis comprises a track for providing mounting guidance to the electronic evaporator.

### BRIEF DESCRIPTION OF THE DRAWINGS

In order to further describe embodiments of the present invention, the embodiments of the present invention will be presented in detail with reference to the accompanying drawings. It should be understood that these accompanying drawings may delineate only typical embodiments of the present invention, and thus will not be considered to limit the scope of protection claimed by the present invention.

In addition, the accompanying drawings show main connection relationships or relative positional relationships between components, but not all of these relationships, and components and connections in the accompanying drawings are not necessarily drawn to actual scale.
FIG. 1 shows a connector applicable to an anesthesia machine system according to an embodiment of the present invention;
FIG. 2A shows an exploded view of a connector according to an embodiment of the present invention;
FIG. 2B shows a perspective view of assembly of the connector according to the embodiment in FIG. 2A;
FIG. 2C shows a side view of assembly of the connector according to the embodiment in FIG. 2A;
FIG. 3 shows a schematic diagram of terminals provided on a first end of a front housing according to an embodiment of the present invention;
FIG. 4A shows a perspective view of a separate second wrapping member of a connector according to an embodiment of the present invention;
FIG. 4B shows a perspective view of a second wrapping member gathering rear ends of electric wires together according to an embodiment of the present invention;
FIG. 5A shows a schematic structural diagram of a front housing and a rear housing of a connector in a separated state according to an embodiment of the present invention;
FIG. 5B shows a schematic structural diagram of a separate rear housing;
FIG. 6 shows a partial enlarged view of a connecting portion between the connector in FIG. 1 and an electronic evaporator;
FIG. 7 shows a schematic diagram of terminals of a connector connected in an interface of an electronic evaporator according to an embodiment of the present invention;
FIG. 8A shows a schematic diagram of a chassis in an anesthesia machine system for connecting a connector to an electronic evaporator according to an embodiment of the present invention;
FIG. 8B shows a schematic diagram of a connector fixed to a chassis according to an embodiment of the present invention; and
FIG. 8C shows a schematic diagram of a connector fixed on a chassis and an electronic evaporator connected to each other in position.

### DETAILED DESCRIPTION

The following detailed description is provided with reference to the accompanying drawings. The accompanying drawings illustrate, via examples, specific embodiments capable of implementing the claimed subject matter. It should be understood that the following specific embodiments are intended to describe typical examples in detail for the purpose of illustration, but should not be understood as limiting the present invention. On the premise of fully understanding the spirit and gist of the present invention, those skilled in the art can make appropriate modifications and adjustments to the disclosed embodiments without departing from the spirit and scope of the claimed subject matter of the present invention.

In the following detailed description, numerous specific details are set forth in order to provide a thorough understanding of the embodiments described. However, it will be apparent to those of ordinary skill in the art that the described various embodiments can be implemented without these specific details. In other examples, commonly-known structures are not described in detail to avoid unnecessarily obscuring aspects of the embodiments. Unless otherwise defined, terms used herein shall have the same meanings as commonly understood by those of ordinary skill in the art to which the present disclosure belongs.

The terms "first", "second", etc., in the description and claims of the present application do not denote any order, quantity, or importance, but are merely intended to distinguish between different constituents or features.

Embodiments of the present application are exemplary implementations or examples. Reference in the description to "embodiments", "one embodiment", "some embodiments", "alternative embodiments", or "other embodiments" means that specific features and structures described with reference to embodiments are included in at least some embodiments of the present technology, but are not necessarily included in all embodiments. Various occurrences of "embodiments", "one embodiment", or "some embodiments" do not necessarily refer to the same embodiment. Elements or aspects from one embodiment may be combined with elements or aspects of another embodiment.

A connector for a medical device, a connector assembly including the connector, and an anesthesia machine system including the connector assembly according to the present invention will be described in detail below with reference to the accompanying drawings.

In the following some embodiments of the present disclosure, a connector of the present invention is described mainly with reference to an anesthesia machine system. However, it should be understood that the present invention is not limited to this. After the design idea of the present invention is understood, the connector of the present invention may be used in any other suitable medical device.

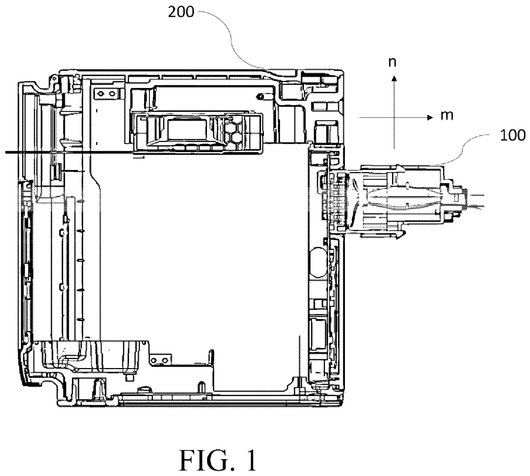

FIG. 1 shows a connector 100 applicable to an anesthesia machine system according to an embodiment of the present invention. The connector 100 and an electronic evaporator 200 are in a connected state. In this embodiment, the connector 100 may be used for connection between an anesthesia machine main unit (not shown) of an anesthesia machine system and the electronic evaporator 200. The anesthesia machine system delivers an anesthetic agent into the body of a subject (e.g., a patient) to anesthetize the patient. The anesthesia machine system may generically include the anesthesia machine main unit (not shown), the electronic evaporator 200, and a gas path for delivering the anesthetic agent into the body of the subject. The anesthetic agent is generally stored in liquid form, and may be first vaporized in the electronic evaporator 200 and then delivered into the body of the subject via the gas path of the anesthesia machine system. In embodiments of the present invention, the anesthesia machine main unit may supply power and transmit information to and control the electronic evaporator 200 by means of the connector 100.

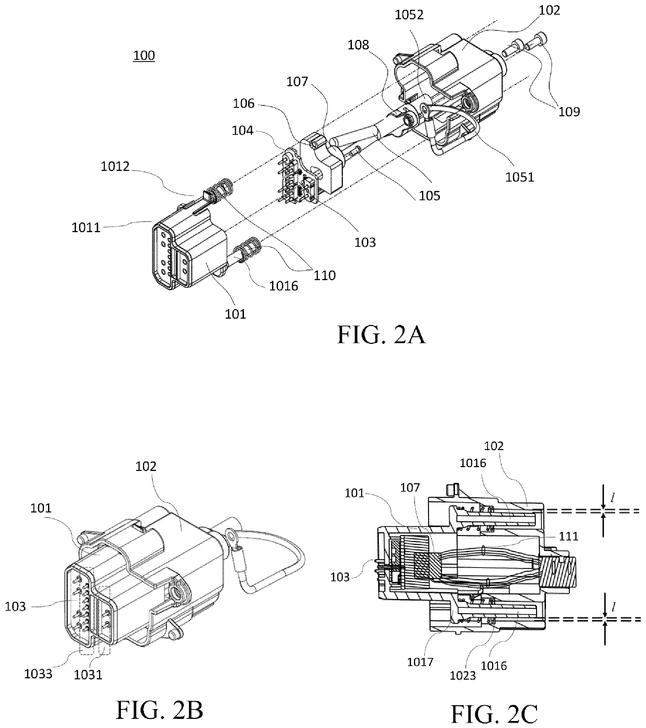

FIG. 2A is an exploded view of the connector 100 according to one embodiment of the present invention. FIG. 2B is a perspective view of assembly of the connector 100 according to the embodiment in FIG. 2A. FIG. 2C is a side view of assembly of the connector 100 according to the embodiment in FIG. 2A. The connector 100 of the present invention can be used for medical devices, such as the anesthesia machine described above in the present application or other medical devices in the art. As shown in FIG. 2A to FIG. 2C, the connector 100 may include a first housing (also referred to as a front housing) 101 and a second housing (also referred to as a rear housing) 102. The front housing 101 may include a first end 1011 and a second end 1012 opposite each other. The second end 1012 of the front housing 101 may be configured to be floatingly connected within the rear housing 102, and the first end 1011 of the front housing 101 may be exposed out of the rear housing 102. The connector 100 of the present invention may further include terminals 103. The terminals 103 may be provided on the first end 1011 of the front housing 101. In embodiments of the present invention, the terminals 103 include a power supply terminal 1031 and a signal terminal 1033. The terminals 103 of the connector 100 may be coupled or connected to a matching interface on the electronic evaporator 200 as shown in FIG. 1. In this way, the electronic evaporator 200 may perform power connection and signal transmission via the terminals 103 of the connector 100.

In the embodiment described above, being floatingly connected means that the front housing 101 floats relative to the rear housing 102 at least in the axial direction (or, the lengthwise direction of the rear housing 102). That is, the front housing 101 is telescopic relative to the rear housing 102. When an electrical connection is established by using the connector 100 in the embodiments of the present application, the floatable connector 100 ensures that a connection process is provided with certain cushioning and is therefore performed more smoothly, which greatly avoids damage to the connector 100. During use, a user does not need to strictly align an interface to be connected (e.g., an interface of a medical device) with the connector 100 at the beginning, and is allowed to have certain inaccuracy. In an aspect, connection efficiency is improved, and in another aspect, the service life of the device is extended. In addition, the terminals 103 of the connector 100 of the present application include both the power supply terminal 1031 and the signal terminal 1033, and the user needs to perform connection only once to provide power and a signal transmission function for a component of a medical device simultaneously. This reduces complexity of operation, and can also conserve space as much as possible for some structurally compact medical devices.

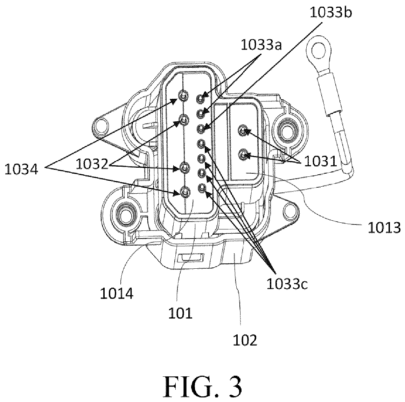

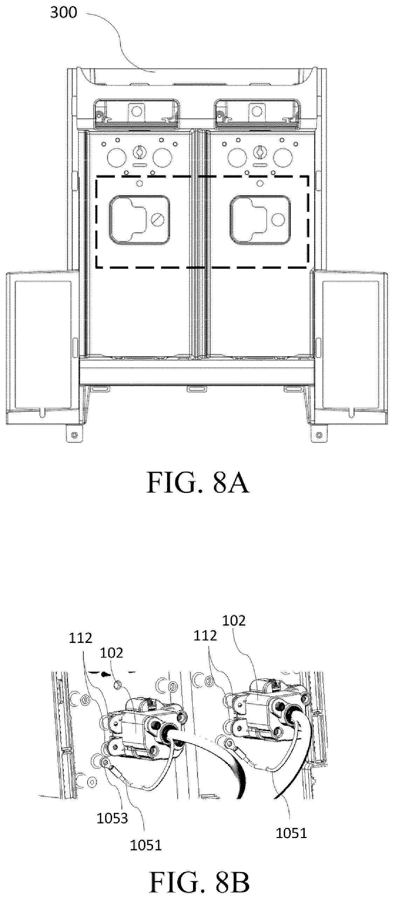

FIG. 3 shows a schematic diagram of the terminals 103 provided on the first end 1011 of the front housing 101 according to an embodiment of the present invention. In one embodiment, the front housing 101 may be made of an electrostatic discharge (ESD) material, so that any tribocharge accumulating on the connector 100 can be dispersed to elsewhere, for example to a chassis 300 of an anesthesia system described in further detail below with reference to FIG. 8A to FIG. 8C. This may add a layer of safety measures to an electrical/oxygen-rich interface. In one specific embodiment, the front housing 101 may include a polyphenyl sulfide - polystyrene (PPS-PA) composite.

As described above, the terminals 103 include the power supply terminal 1031 and the signal terminal 1033. In addition, the terminals 103 may further include a power return terminal 1032. The power return terminal 1032 may provide a power return path. In one preferred embodiment, the terminals 103 may include at least two power supply terminals 1031 and at least two power return terminals 1032 respectively corresponding thereto. Each power supply terminal 1031 and each corresponding power return terminal 1032 may form a power supply loop. It is advantageous to provide at least two sets of power supply terminals 1031 and power return terminals 1032, and redundancy can be provided when one set is damaged. In one embodiment, the power supply terminal 1031 may be configured to provide direct current power supply to the connected medical device (e.g., the electronic evaporator 200).

In embodiments of the present invention, the terminals 103 may further additionally include a ground terminal 1034 for grounding the medical device (e.g., the electronic evaporator 200) connected to the connector 100. In one embodiment, at least two ground terminals 1034 may be included so as to provide redundancy when one of the ground terminals 1034 is contaminated or damaged.

In embodiments of the present invention, the signal terminals 1033 may include a CAN bus terminal 1033a and/or an Ethernet terminal 1033c. The CAN bus terminal 1033a may be configured to transmit a signal to the medical device connected to the connector 100, for example, transmitting a signal from the anesthesia machine main unit to the electronic evaporator 200. The Ethernet terminal 1033c may be configured to provide an Ethernet connection to the connected medical device (e.g., the electronic evaporator 200 described above). In addition, the signal terminals 1033 may further include a test terminal 1033b.

In a preferred embodiment of the present invention, the lengths of at least some signal terminals 1033 exposed at the first end 1011 of the front housing 101 are less than the length of the power supply terminal 1031 exposed at the first end 1011. For example, the length of at least the test terminal 1033b in the signal terminals 1033 exposed at the first end 1011 of the front housing 101 is less than the length of the power supply terminal 1031 exposed at the first end 1011, thereby preventing an electric arc from occurring when the connector 100 is connected or disconnected. Specifically, the test terminal 1033b may be configured to control, according to the electrical connection status thereof, energization and deenergization of the power supply terminal 1031, and the length difference between the power supply terminal 1031 and the test terminal 1033b can cause electrical connection of the power supply terminal 1031 to be earlier than electrical connection of the test terminal 1033b, and can cause removal of the electrical connection of the power supply terminal 1031 to be later than removal of the electrical connection of the test terminal 1033b. In other words, when the connector 100 is connected to the medical device to be connected, the power supply terminal 1031 that is longer can be connected first, at which point the power supply terminal 1031 is not energized, and then the test terminal 1033b that is shorter is connected to trigger energization of the power supply terminal. When the connector 100 is disconnected from the connected medical device, the test terminal 1033b that is shorter can be disconnected first, so that before the power supply terminal 1031 that is longer is disconnected, the test terminal 1033b can be disconnected to trigger immediate disabling of the energization of the power supply terminal 1031. In this way, the chance of spark production can be eliminated.

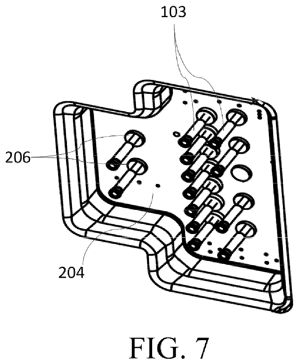

As one specific example, in embodiments where, for example, the connector 100 is configured for connection between the anesthesia machine main unit and the electronic evaporator 200, the anesthesia machine main unit may include a power source for supplying power to the power supply terminal 1031 of the connector 100 and a power management circuit. The power management circuit can be configured to, when a test signal generated by connection between the test terminal 1033b in the signal terminals 1033 and a corresponding position in an interface (for example, an interface 204 shown in FIG. 7 to be described below) of the electronic evaporator 200 is detected, control the power source to supply power to the power supply terminal 1031 of the connector 100. For example, the power management circuit may control, by turning on a control switch, the power source to supply power to the power supply terminal 1031 of the connector 100. As described above, the test terminal 1033b is shorter than the power supply terminal 1031, so that when the connector 100 is connected, the test terminal 1033b can turn on the power source after the power supply terminal 1031 is connected in position, so as to supply power to the power supply terminal 1031. The power management circuit may further be configured to, when the test signal is not detected, control the power source to stop supplying power to the power supply terminal 1031 of the connector 100. As described above, the test terminal 1033b is shorter than the power supply terminal 1031, so that when the connector 100 is disconnected, the test terminal 1033b can be disconnected earlier than the power supply terminal 1031, or power supply to the power supply terminal 1031 can be stopped before the power supply terminal 1031 is disconnected. For example, the power management circuit may perform control by turning off the switch to stop supplying power to the power supply terminal 1031 of the connector 100. In this way, spark production can be suppressed.

It should be noted that supplying power to the power supply terminal 1031 of the connector 100 should be understood as providing a normal working voltage (i.e., a strong current) to the connected, e.g., electronic evaporator 200. When the connector 100 is connected so that the power supply terminal 1031 is connected but the test terminal 1033b is not connected, or when the connector 100 is disconnected so that the test terminal 1033b is disconnected but the power supply terminal 1031 is not disconnected, it may be allowed in some embodiments that actually, a weak current still passes through the power supply terminal 1031 although the a power control circuit stops supplying a strong current to the electronic evaporator 200 because the test signal is not detected. The weak current can be used to perform basic functions such as testing an electrical connection status of the terminal, without resulting in hazards such as electric sparks, etc.

In a preferred embodiment, the power management circuit of the anesthesia machine main unit may further be configured to: after the test signal is detected and lasts a certain preset time, control the power source to supply power to the power supply terminal 1031. The power management circuit may activate a timer immediately after the test signal is detected, and control, after the timer reaches a preset time, the power source to supply power to the power supply terminal 1031. The preset time may be a time length that is set as desired. In one embodiment, the preset time may be set to be less than five seconds. In further embodiments, the preset time may be set to be less than three seconds. In still further embodiments, the preset time may be set to be two seconds. In addition, the power management circuit of the anesthesia machine main unit may further be configured to, when the test signal is not detected, immediately control the power source to stop supplying power to the power supply terminal 1031. In this way, production of electric sparks can be more effectively prevented.

In some embodiment, the lengths of all the signal terminals 1033 exposed at the first end 1011 of the front housing 101 may be set to be less than the length of the power supply terminal 1031 exposed at the first end 1011. In some other embodiments, only the length of the test terminal 1033b exposed at the first end 1011 of the front housing 101 may be set to be less than the length of the power supply terminal 1031 exposed at the first end 1011, and the lengths of the other signal terminals 1033 may be set to be the same as the length of the power supply terminal 1031. In embodiments where only the length of the test terminal 1033b is less than the length of the power supply terminal 1031, the other signal terminals 1033 can be sufficiently compressed when connected to the corresponding interface (e.g., the interface 204 in FIG. 7), thereby ensuring good contact.

In some other preferred embodiments, as shown in FIG. 3, the front housing 101 may include a first portion 1013 and a second portion 1014. The first portion 1013 and the second portion 1014 may be different in at least one among shape or size. The first portion 1013 and the second portion 1014 of the front housing 101 are different in shape and/or size, so that hazards and damage to the device caused by wrong insertion can be prevented during use of the connector 100. It can be understood that the first portion 1013 and the second portion 1014 described above refer to two portions resulting from segmentation performed by a cutting plane parallel to a lengthwise direction of the front housing 101 (or, a direction of a connecting line between the first end and the second end). In such configurations, the first end and the second end of the same front housing 101 both have cross sections different in shape or size. Therefore, it can be ensured that an effective fool-proof effect can be achieved no matter during assembly with the rear housing 102 or during electrical connection to a component outside the connector.

It should be noted that the above description of the two portions is merely used to describe the shape of the front housing 101, but does not represent a definition of a manufacturing process. For example, the entire front housing 101 may be integrally injection-molded.

In some embodiments. The power supply terminal 1031 may be provided in the first portion 1013 of the front housing 101, and the signal terminal 1033 may be provided in the second portion 1014 of the front housing 101. Wiring of the power supply terminal and wiring of the signal terminal are performed separately, thereby facilitating assembly. However, the present application does not make a unique definition.

It should be understood that, as an example, FIG. 3 schematically shows different types of terminals 103, the number of terminals 103 of each type, and a specific arrangement of the terminals 103. However, this is merely an example, but is not restrictive. In some other embodiments of the present invention, more or fewer types of terminals 103 may be included. For example, the terminals 103 of the present invention may include the power supply terminal 1031 and the signal terminal 1033, and the signal terminal 133 may include one or a plurality of the CAN bus terminal 1033a, the test terminal 1033b, or the Ethernet terminal 1033c. In addition, in some other embodiments of the present invention, the number of terminals 103 of each type and the arrangement of the terminals 103 may also be selected and set according to specific requirements, and for example, may be correspondingly set according to circuit design of the medical device (e.g., the anesthesia machine main unit and the electronic evaporation tank 200 described above) involved.

In the present invention, the same connector 100 includes the power supply terminal 1031 and the signal terminal 1033 respectively used for power connection of the medical device (e.g., the electronic evaporator 200) and information transmission, and in addition, the connector 100 may further additionally include the ground terminal 1034, thereby improving the integration level of the connector 100, reducing use of different types of connectors, and allowing the device to be used easily and conveniently. In addition, the power supply terminal 1031 and the signal terminal 1033 in the present invention may be arranged in regions having different shapes and/or sizes, so that wrong insertion and connection of the connector 100 can be effectively prevented, thereby improving the safety of using the connector 100. Further, the signal terminals 1033 of the present invention may include the test terminal 1033b, and the length of the test terminal 1033b exposed at the first end 1011 of the front housing 101 is set to be less than the length of the power supply terminal 1031 exposed at the first end 1011, so that such a length difference can cause electrical connection of the power supply terminal 1031 to be earlier than electrical connection of the test terminal 1033b, and can cause removal of the electrical connection of the power supply terminal 1031 to be later than removal of the electrical connection of the test terminal 1033b, and therefore the test terminal 1033b can be used to control, according to the electrical connection status thereof, energization and deenergization of the power supply terminal 1031. This eliminates the possibility of production of electric sparks, thereby further improving the safety of using the connector 100.

The inventor found during actual use that a floating connector in use may encounter great resistance and that in some scenarios, a floating connector (e.g., the front housing 101) is prone to tilt and shift during compression. Further, the inventor found that the phenomena described above are caused by a cable in the housing of the connector. A combined cable typically has high stiffness. When the connector is being compressed, the cable produces resistance to compression, and the resistance of the cable does not act perpendicularly on the front housing of the connector during the compression. This affects a floating effect of the connector. In view of this, improvements are provided in some embodiments of the present application.

Returning to FIG. 2A, the connector 100 may further include a circuit board 104. The circuit board 104 may be accommodated in the front housing 101. In one embodiment, the circuit board 104 may be fixed to the first end 1011 of the front housing 101 by means of one or more screws (not shown). The terminals 103 may be fixed on a first side of the circuit board 104, and pass through the first end 1011 of the front housing 101, for example, passing through a hole on the first end 1011 of the front housing 101. A second side of the circuit board 104 may include a plurality of electric wires 105 coupled to the terminals 103. In embodiments of the present invention, the plurality of electric wires 105 may be divided into at least two cables, and the electric wires 105 in each cable may be bundled via a bundling member 111 (as shown in FIG. 2C). As an example, the bundling member 111 may include, but is not limited to, a clip, a tie, or the like.

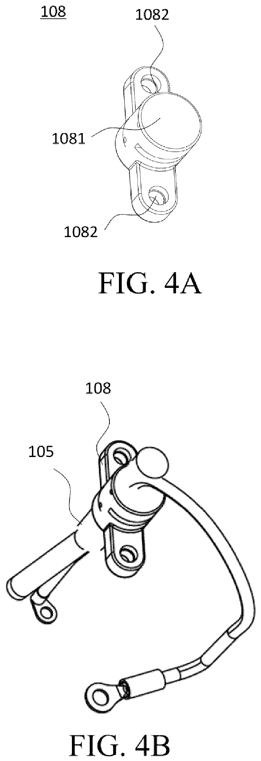

In such a manner, the plurality of electric wires 105 are no longer combined into one cable in the connector, but are divided into at least two cables. In contrast to one cable, the at least two cables deform more easily under the action of an external force, thereby reducing resistance to floating of the front housing 101. In addition, the at least two cables can provide, at more sites, support for the front housing, so that the front housing is subjected to a force more uniformly. In embodiments of the present invention, a first end (also referred to as a front end) of the electric wire 105 close to the terminals 103 is fixed in the front housing 101, and a second end (also referred to as a rear end) of the electric wire 105 located away from the terminals 103 is fixed in the rear housing 102. For example, in one specific embodiment, the connector 100 may further include a first wrapping member 106 provided in the front housing 101. The first wrapping member 106 may be configured to gather the front ends of the electric wires 105 together, and may fix the front ends of the electric wires 105 in the front housing 101 via glue (as shown in FIG. 2C). The connector 100 may further include a second wrapping member 108. FIG. 4A shows a perspective view of the separate second wrapping member 108 of the connector 100 according to an embodiment of the present invention. FIG. 4B shows a perspective view of the second wrapping member 108 gathering the rear ends of the electric wires 105 together according to an embodiment of the present invention. As shown in FIG. 2A, the second wrapping member 108 may be provided and fixed in the rear housing 102. The second wrapping member 108 may be configured to gather the rear ends of the electric wires 105 together. For example, the second wrapping member 108 may include a bundling hole 1081 in the middle to gather the rear ends of the electric wires 105 together. In addition, the second wrapping member 108 may fix the rear ends of the electric wires 105 in the rear housing 102 by means of a fastener 109. As an example, the fastener 109 may include a screw, and the second wrapping member 108 may include one or more side holes 1082 so as to allow the screw to pass through the side hole 1082 and to fix the second wrapping member 108 in the rear housing 102 by means of the screw. The rear housing 102 may include a screw hole that mates with the screw, so as to fix the rear end of the electric wire 105 in the rear housing 102 by screwing the screw in the screw hole. Preferably, the fastener 109 may include at least two screws, and correspondingly, at least two side holes 1082 are included.

As described above, the terminals 103 may include the ground terminal 1034. Correspondingly, as shown in FIG. 2A, the electric wires 105 may include a ground wire 1051 coupled to the ground terminal 1034. After bundling is performed via the second wrapping member 108, the ground wire 1051 may be separated from the other electric wires 1052. The ground wire 1051 may be connected to a ground structure, e.g., the chassis 300 of the anesthesia machine system to be described below, so that the medical device (e.g., the electronic evaporator 200) is connected to ground.

In embodiments of the present invention, as described above, the second end 1012 of the front housing 101 may be configured to be floatingly connected within the rear housing 102, and the first end 1011 of the front housing 101 may be exposed out of the rear housing 102. According to any embodiment described above, being floatingly connected includes axial floating of the front housing 101 (the first housing) relative to the rear housing 102 (the second housing). In other embodiments of the present application, being floatingly connected may further include circumferential floating of the front housing 101 relative to the rear housing 102, which will be described in detail below.

Specifically, as shown in FIG. 2C, a gap I is present between the front housing 101 and the rear housing 102. The presence of the gap I enables the second end 1012 of the front housing 101 to float circumferentially relative to the rear housing 102. Such a design of the floating structure of the present invention is advantageous. When tolerance accumulates to prevent the connector 100 from entering the interface (e.g., the interface 204) in the connected medical device (e.g., the electronic evaporator 200) smoothly, floating can compensate for tolerance accumulation, and help the connector 100 to enter the interface smoothly. In one embodiment, the gap *l* may be set to be 0.5 millimeters to 3 millimeters. In further embodiments, the gap *l* may be set to be about 1 millimeter.

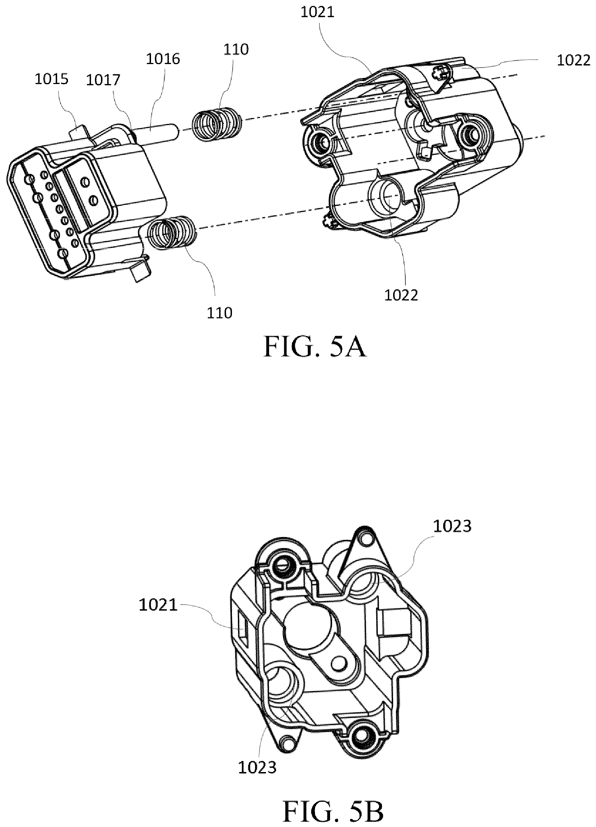

FIG. 5A shows a structural diagram of the front housing 101 and the rear housing 102 of the connector 100 in a separated state. FIG. 5B shows a schematic structural diagram of the separate rear housing 102. In embodiments of the present invention, a protrusion 1015 may be provided on a side wall of the front housing 101, and a notch 1021 may be provided in a position on a side wall of the rear housing 102 opposite the protrusion 1015. The notch 1021 may be used to accommodate the protrusion 1015. The notch 1021 is larger than the protrusion 1015, thereby at least partially defining a floating range of the front housing 101 relative to the rear housing 102. In one implementation, the protrusion 1015 may be formed to have the shape of a hook.

The circumferential direction described above may be understood as the direction of an inner circumference of the rear housing 102, and is defined collectively by an inner surface of the rear housing 102 and the gap *l* between the front housing and the rear housing. The circumferential floating can compensate for the tolerance and reduce device precision requirements, and more importantly can reduce requirements on precision of connection between the medical device and the connector 100. In the case that the interface of the medical device is not strictly aligned with the connector 100, the front housing 101 of the connector 100 can therefore bend by a certain degree, thereby achieving a cushioning effect.

It should be noted that under the teaching of the present disclosure, the connector 100 may float in a plurality of manners. For example, the connector 100 may float axially (i.e., telescopic). Alternatively, the connector 100 may float circumferentially (i.e., the front housing is movable within the range of the inner circumference of the rear housing). In a preferred embodiment, the connector 100 may have both the axial floating function and the circumferential floating function. The technical effect of being floatingly connected is described in detail above and will not be described herein again.

In embodiments of the present invention, the length of the electric wire 105 located inside the connector 100 should be set so that when the protrusion 1015 moves to an edge of the notch 1021, the electric wire 105 is not pulled. In other words, a certain length needs to be reserved for the electric wire 105 inside the connector 100, so that the axial floating range of the front housing 101 relative to the rear housing 102 is not limited by the length of the electric wire 105, but is limited by the range within which the protrusion 1015 can float axially in the notch 1021.

The second end 1012 of the front housing 101 may include a guide pin 1016 extending from the second end 1012. Correspondingly, the rear housing 102 may include a hole 1022 for accommodating the guide pin 1016. The connector 100 may further include a spring 110. The spring 110 may be sleeved on the guide pin 1016 of the front housing 101 and accommodated in the hole 1022 of the rear housing, so as to assist the front housing 101 in floating circumferentially/axially relative to the rear housing 102.

In embodiments of the present invention, the radial dimension of a base 1017 of the guide pin 1016 may be set to be greater than the radial dimension of the spring 110 (see FIG. 2C and FIG. 5A), so that the spring 110 can abut against the base 1017 of the guide pin 1016 to restrict one side of the spring 100. As shown in 5B, the interior of the hole 1022 may include a step 1023 for abutting against the spring 110 to restrict the other side of the spring 100. Therefore, the spring 110 can be restricted in a particular space inside the connector 100.

In the assembled connector 100, the spring 110 may be pre-compressed by a certain amount. When the connector 100 is connected to the medical device (e.g., the electronic evaporator 200), the front housing 101 provided with the terminals 103 is subjected to a force when contacting the medical device, and therefore floats axially, i.e., moving backwards (e.g., moving in a direction m shown in FIG. 2A). In this case, the compressed spring 100 can provide a pushing force in an opposite direction (e.g., a pushing force opposite to the direction m), so that a securing force can be provided to maintain stable and reliable contact between the terminals 103 and the corresponding interface 204 (e.g., corresponding pads 206 in the interface 204) of the electronic evaporator 200. In addition, the spring 110 may also help the front housing 101 to move more smoothly relative to the rear housing 102.

Preferably, the connector 100 of the present invention may include a group of guide pins 1016 and a group of corresponding springs 110 provided on a diagonal line of a cross section of the rear end 1012 of the front housing 101 of the connector 100, and a group of corresponding holes 1022 provided in the rear housing 102, so that the circumferential/axial floating of the front housing 101 relative to the rear housing 102 is more stable and uniform. In addition, such design of the present invention may further allow the length of the guide pin 1016 extending from the rear end 1012 of the front housing 101 and a corresponding depth of the hole 1022 in the rear housing 102 to be designed according to actual requirements, so that the axial floating of the front housing 101 relative to the rear housing 102 can have a longer guide path in the present invention.

It is further recognized by the inventor that the floating connector may damage a circuit inside the connector. For example, the floating connector incurs shear stress to the terminals and/or the electric wires during deformation, resulting in undesirable poor contact of the connector, or even disconnection. In addition, such tilt bends or damages the terminals, thereby shortening the service life of the connector. However, in the present invention, the technical solution described above can solve the above problem to a great extent by dividing the electric wires 105 into bundles. The reason is that the electric wires divided into bundles have higher flexibility, and deform more uniformly when subjected to a force. In some other embodiments, improvements to the floating connector are further made in the present application. Further detailed description is provided below.

In embodiments of the present invention, the terminals 103 may be fixed to the front housing 101 by means of, for example, the circuit board 104. The front ends of the electric wires 105 are fixed to the front housing 101, and the rear ends of the electric wires 105 are fixed to the rear housing 102. The front housing 101 is floatingly mounted to the rear housing 102, so that movement of the rear housing 105 and the electric wires 105 is not transmit to the front housing 101, and is therefore not transmitted to the terminals 103 fixed to the front housing 101, thereby improving the stability and reliability of connection of the terminals 103.

Specifically, when the connector 100 is connected to the medical device (e.g., the electronic evaporator 200), the front housing 101 provided with the terminals 103 is subjected to a backward force (e.g., a force in the direction m shown in FIG. 1) when contacting the medical device, and therefore moves backwards. In this case, the inside electric wires 105 are pressed, and therefore bend and deform. In the solution of the present invention, as described above, one end of the electric wire 105 inside the connector 100 may be fixed in the front housing 101 by means of, for example, the first wrapping member 106, and the other end of the electric wire 105 may be fixed in the rear housing 102 by means of, for example, the second wrapping member 108. In addition, the electric wires 105 may be divided into at least two cables, and the electric wires 105 in each cable may be bundled by using the bundling member 111, thereby improving flexibility. Therefore, no matter how a middle portion of the electric wire 105 bends, deforms, or moves inside the housing, the front housing 101 provided with the terminals 103 can still remain inserted and connected stably without being affected thereby, so that the connector 100 is well and stably connected to the connected medical device (e.g., the electronic evaporator 200) during operation. This can reduce the risk that the connector 100 is disconnected during operation, thereby ensuring normal operation of the connected medical device (e.g., the electronic evaporator 200).

In addition, in the design of the present invention, although the front housing 101 and the rear housing 102 float relative to each other, the rear ends of the electric wires 105 are fixedly connected to the rear housing 102. Correspondingly, the gravitational force resulting from the weight of the electric wires 105 borne on the rear housing 102 and a pulling force resulting from movement are not transmitted to the front housing 101 either, so that the front housing 101 is not tilt downwards, and the connector 100 can achieve smooth connection and disconnection. Further, the front housing 101 is stably connected and therefore does not tilt downwards, so that no shear stress is present on the terminals 103, and the terminals 103 can always be perpendicularly connected to the corresponding interface (e.g., the interface 204), thereby extending the service life of the terminals 103.

In embodiments of the present invention, the floating range of the front housing 101 relative to the rear housing 102 may be defined by means of the protrusion 1015 on the side wall of the front housing 101 and the notch 1021 correspondingly provided on the side wall of the rear housing 102. The size of the notch 1021 may be designed flexibly, thereby adjusting the floating range of the front housing 101 relative to the rear housing 102. In embodiments of the present invention, floating can be achieved in up to six dimensions (including the axial and circumferential floating described above), for example, the direction m and a direction opposite thereto, a direction n and a direction opposite thereto, and two opposite directions perpendicular to a plane where m and n are located. Therefore, the technical solution of the present invention further allows the connector 100 to float more. This can compensate for misalignment between the connector 100 and the interface of the medical device (e.g., the electronic evaporator 200) to be connected caused by tolerance. In turn, since the connector 100 of the present invention can absorb some accumulated tolerance, requirements on machining precision of relevant parts can be reduced accordingly, thereby reducing production difficulty, improving productivity, and reducing costs.

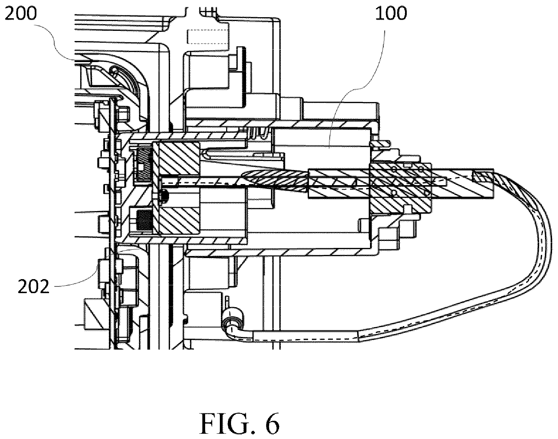

Returning to the specific embodiments where the connector 100 is used for the anesthesia machine system and referring to FIG. 6, FIG. 6 shows a partial enlarged view of a connecting portion between the connector 100 in FIG. 1 and the electronic evaporator 200. In embodiments where the connector 100 is used to connect the electronic evaporator 200, a beveled surface may be provided around an inner surface of the interface of the electronic evaporator 200 connected to the connector 100. When contacting the beveled surface 202, the first end 1011 of the front housing 101 can float to guide the interface 204 of the electronic evaporator 200 to be connected to the connector 100, thereby more smoothly connecting the connector 100 to the interface 204 of the electronic evaporator 200.

FIG. 7 shows a schematic diagram of the terminals 103 of the connector 100 connected within the interface 204 of the electronic evaporator 200 according to an embodiment of the present invention. As one example but not limitation, the terminals 103 of the connector 100 may include pogo pins, and correspondingly, the interface 204 of the electronic evaporator 200 may include pads 206 in one-to-one correspondence with the pogo pins.

FIG. 8A shows a schematic structural diagram of a chassis 300 in an anesthesia machine system for connecting the connector 100 to the electronic evaporator 200 according to an embodiment of the present invention. FIG. 8B shows a schematic structural diagram of the connector 100 fixed to the chassis 300. A dashed box in FIG. 8A shows positions on the chassis 300 for mounting two connectors 100. In embodiments of the present invention, the second housing 102 of the one or more connectors 100 may be fixed to the position on the chassis 300. For example, the second housing 102 of the connector 100 may be fixed to the chassis 300 by means of a fastener 112 such as a screw or the like. The chassis 300 may include a metal material. As described above, the ground wire 1051 of the connector 100 may also be fixed to the chassis 300. For example, an end portion of the ground wire 1051 may have a hole 1053, so that the end portion of the ground wire 1051 may be fixed to the chassis 300 by using a screw.

As an example, FIG. 8A and FIG. 8B show the two connectors 100 and the positions on the chassis 300 for receiving the two connectors 100. However, it can be understood that in other embodiments of the present invention, only one or more than two connectors 100 may be fixed to the chassis 300. Correspondingly, the chassis 300 may include only one or more than two mounting positions.

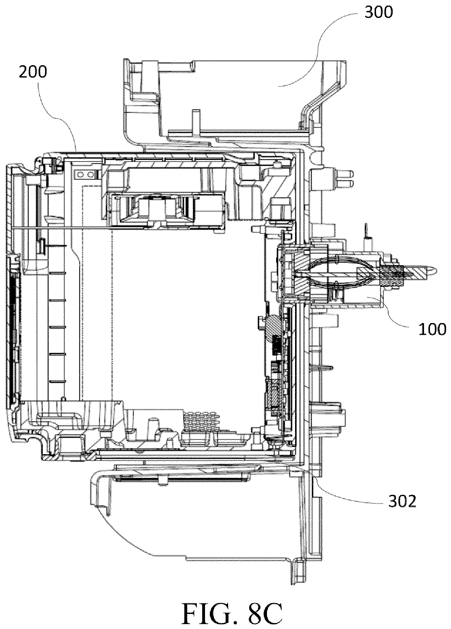

The chassis 300 can be used to connect the connector 100 fixed thereon to the electronic evaporator 200. Specifically, FIG. 8C shows a schematic diagram of the connector 100 fixed on the chassis 300 and the electronic evaporator 200 connected to each other in position. The bottom of the chassis 300 may include a track 302. The track 302 may be used to provide a mounting guide to the electronic evaporator 200, so that a bottom surface of the electronic evaporator 200 can move along the track 302 into the chassis 300 by means of a sliding/wiping action, thereby approaching the front end 1011 of the front housing 101 of the connector 100, and assisting the interface 204 on the electronic evaporator 200 in being connected to the terminals 103 on the front housing 101 of the connector 100.

It should be understood that the description of the positions and directions in the present description is provided with reference to specific embodiments shown in the accompanying drawings, and is therefore a relative position description. In other embodiments where the placement direction of the device and apparatus is opposite or different than the direction shown in the drawings, these position descriptions may vary accordingly.

Therefore, person skilled in the art can made appropriate modifications and adjustments to the embodiments described in detail above without departing from the spirit and gist of the present invention Therefore, it is intended that the claimed subject matter is not limited to only particular examples disclosed, and the claimed subject matter may also include all implementations that fall within the scope of the appended claims and equivalents thereof.

## Claims

1. A connector for a medical device, the connector comprising:
a first housing, comprising a first end and a second end opposite each other;
a second housing, the second end of the first housing being configured to be floatingly connected within the second housing, and the first end being exposed out of the second housing; and
terminals, provided on the first end of the first housing, the terminals comprising a power supply terminal and a signal terminal.

2. The connector according to claim 1, wherein the lengths of at least some signal terminals exposed at the first end are less than the length of the power supply terminal exposed at the first end.

3. The connector according to claim 2, wherein the at least some signal terminals comprise a test terminal, the test terminal being configured to control, according to the electrical connection status thereof, energization and deenergization of the power supply terminal, and the length difference causing electrical connection of the power supply terminal to be earlier than electrical connection of the test terminal, and causing removal of the electrical connection of the power supply terminal to be later than removal of the electrical connection of the test terminal.

4. The connector according to claim 2 or 3, wherein the signal terminals comprise:
a CAN bus terminal, configured to transmit a signal to a medical device connected to the connector; and/or
an Ethernet terminal, configured to provide an Ethernet connection to the connected medical device.

5. The connector according to claim 1, wherein the first housing comprises a first portion and a second portion, and the first portion and the second portion are different in at least one among shape or size.

6. The connector according to claim 1, wherein the power supply terminals comprise at least two power supply terminals, and the terminals further comprise at least two power return terminals, wherein each of the power supply terminals corresponds to each of the power return terminals to form a power supply loop.

7. The connector according to claim 1, wherein the terminals further comprise a ground terminal.

8. The connector according to claim 1, wherein
the connector further comprises a circuit board accommodated in the first housing, the terminals being fixed on a first side of the circuit board and passing through the first end of the first housing, and a second side of the circuit board comprising a plurality of electric wires coupled to the terminals, wherein the plurality of electric wires are divided into at least two cables.

9. The connector according to claim 8, wherein
the electric wires in each of the at least two cables are bundled via a bundling member.

10. The connector according to claim 8, wherein a first end of the electric wire close to the terminals is fixed in the first housing, and a second end of the electric wire located away from the terminals is fixed in the second housing.

11. The connector according to claim 10, further comprising:
a first wrapping member, provided in the first housing, and configured to gather the first ends of the electric wires together, and fix the first ends of the electric wires in the first housing via glue; and
a second wrapping member, provided in the second housing, and configured to gather the second ends of the electric wires together, and fix the second ends of the electric wires in the second housing via a fastener.

12. The connector according to claim 1, wherein
a gap is present between the first housing and the second housing to allow for circumferential floating of the first housing relative to the second housing, and
a protrusion is provided on a side wall of the first housing, a notch being provided at a position on a side wall of the second housing opposite the protrusion, the notch accommodating the protrusion, and the notch being larger than the protrusion, thereby at least partially defining the floating range of the first housing relative to the second housing.

13. The connector according to claim 1, wherein
the second end of the first housing comprises a guide pin extending from the second end,
the second housing comprises a hole for accommodating the guide pin, and
the connector further comprises a spring, the spring being sleeved on the guide pin of the first housing and being accommodated in the hole of the second housing, so as to assist the first housing in axial floating relative to the second housing.

14. The connector according to claim 13, wherein
the radial dimension of a base of the guide pin is greater than the radial dimension of the spring, and the interior of the hole comprises a step for abutting against the spring to restrict the spring in a particular space.

15. A connector assembly, the connector assembly comprising:
the connector according to any one of claims 1-14, and
an electronic evaporator, comprising an interface matching the connector, wherein the electronic evaporator performs power and signal transmission via the terminals of the connector.
